# FASCICULE DE BREVET EUROPEEN

(11) **EP 0 498 728 B1**
(45) Date de publication et mention de la délivrance du brevet: **16.08.1995**
(21) Numéro de dépôt: 92400305.6
(22) Date de dépôt: 06.02.1992
(51) Int. Cl.: C07K 5/062, A61K 7/48

(54) **Amides de la glycyl-sérine et compositions cosmétiques, pharmaceutiques ou alimentaires les contenants**
Glycyl-Serine Amide und diese enthaltende kosmetische und pharmazeutische Zusammensetzungen oder Röhrmittel
Glycyl-serine amides and cosmetic, pharmaceutical or alimentary compositions containting them

(30) Priorité: 06.02.1991 FR 9101308
(43) Date de publication de la demande: 12.08.1992
(73) Titulaire: L'OREAL, 75008 Paris (FR)
(72) Inventeur: Philippe, Michel, F-92160 Antony (FR)
(74) Mandataire: Tonnellier, Jean-Claude

(56) Documents cités:
- EP-A- 0 054 435
- PATENT ABSTRACTS OF JAPAN, vol. 10, no. 303 (C-378)[2359], 16 Octobre 1986,& JP-A-61 118 307 (ICHIMARU FUARUKOSU K.K.) 05-06-1986

## Description

Utilisation d'amides dipeptidiques dérivant de la glycyl-sérine, comme agents tensioactifs ou comme agents hydratants, notamment dans des compositions cosmétiques, pharmaceutiques ou alimentaires, et nouveaux amides dipeptidiques.

La présente invention a pour objet l'utilisation comme agents tensioactifs ou comme agents hydratants, notamment dans des compositions cosmétiques, hygiéniques, pharmaceutiques ou alimentaires, de certains amides dipeptidiques dérivant de la glycyl-sérine.

On connaissait déjà des amides dipeptidiques dérivant de la glycyl-glycine et leur utilisation comme agents tensioactifs ; voir la demande de brevet JP 84994/1984.

On connaissait par ailleurs la N-tétradécanoyl-glycyl-sérine qui a été décrite en tant qu'inhibiteur de prolifération cellulaire dans la demande de brevet JP 146851/1988.

On a maintenant découvert que, de façon surprenante, des amides dérivant de la glycyl-sérine ont des propriétés tensioactives notablement supérieures à celles des amides correspondants dérivant de la glycyl-glycine.

En outre certains amides dérivant de la glycyl-sérine ont un effet hydratant lorsqu'ils sont présents dans des compositions dermopharmaceutiques ou dans des compositions cosmétiques pour la peau.

L'invention a donc pour objet l'utilisation, comme agents tensioactifs et/ou comme agents hydratants, des amides dipeptidiques de formule générale (I)

RCO - NHCH₂CONH - CH(CH₂OH) - COOH (I)

dans laquelle R représente un groupement alkyle, linéaire ou ramifié, éventuellement insaturé, ayant de 7 à 17 atomes de carbone,
ainsi que des sels des composés de formule (I), et des mélanges des composés de formule (I) et/ou de leurs sels.

Les composés de formule I peuvent dériver de la D-,L- ou D,L-sérine.

Parmi les composés de formule (I) utilisables selon l'invention, on citera en particulier ceux pour lesquels R représente un radical alcoyle saturé, et ceux pour lesquels R représente un groupement alcoyle comportant de 1 à 3 double(s) liaison(s) éthylénique(s). De préférence R possède de 7 à 17 atomes de carbone. Le groupement R est choisi par exemple de façon que RCO- représente un groupement octanoyle, décanoyle, dodécanoyle, tétradécanoyle, oléoyle, linoléoyle, etc.

Parmi les sels des composés de formule I (carboxylates), on citera notamment les sels compatibles avec l'application recherchée, c'est-à-dire, selon les cas, avec l'administration par voie orale, ou avec l'application sur la peau ou les muqueuses. Les sels compatibles avec ces diverses applications sont connus, ou peuvent être facilement déterminés par des expériences de routine - par exemple, les sels compatibles avec l'application sur la peau ou les muqueuses sont notamment les sels métalliques tels que les sels de sodium, de zinc, de magnésium, les sels d'aluminium et les sels cuivriques ou les sels de cations organiques tels que les sels d'un ammonium quaternaire de formule Ia : 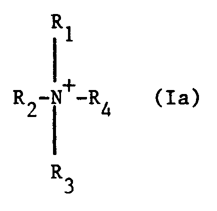
dans laquelle R₁, R₂, R₃ et R₄ représentent indépendamment un groupement -CH₃, -CH₂-C₆H₅ ou -CH₂-CH₂OH.

Par convention, l'expression "composés" ou "dérivés" de formule I désignera ci-après les composés de formule I et/ou leurs sels.

L'invention a donc notamment pour objet l'utilisation des composés de formule I, et de leurs sels, comme agents tensioactifs, en particulier dans des compositions cosmétiques, hygiéniques ou dermopharmaceutiques, ou encore dans des compositions détergentes en général, par exemple des détergents ménagers. Les composés de formule I et leurs sels possèdent notamment un pouvoir moussant très important, et un pouvoir détergent élevé, supérieur à celui de la N-dodécanoyl-glycyl-glycine décrite dans le brevet JP-84994/1984, comme cela est montré dans la partie expérimentale ci-après.

Les composés de formule I sont utilisables comme détergents dans des milieux ayant un pH de 7 à 13 et en particulier de 7 à 9.

Ils peuvent être utilisés notamment comme détergents doux dans des compositions présentant un pouvoir moussant, (compositions cosmétiques, hygiéniques ou pharmaceutiques pour la peau ou les cheveux, ou pour l'hygiène bucco-dentaire ou encore comme émulsifiants dans des préparations alimentaires.

Certains composés de formule I et leurs sels peuvent aussi être utilise's chez l'homme comme agents d'hydratation de la peau, capables notamment de diminuer la perte en eau de la peau. Ces composés, par exemple la N-oléoyl glycyl-(D,L) sérine, permettent dort notamment de conserver ou de restaurer la souplesse de la peau, son élasticité et sa fonction de barrière à l'entrée des substances toxiques. On sait que les compositions cosmétiques ou dermopharmaceutiques destinées à hydrater la peau (préparations hydratantes) sont utilisées chez les personnes ayant une peau dite sèche. Ce phénomène est caractérisé généralement par une peau ayant un taux d'évaporation nettement plus élevé que celui d'une peau saine, par une perte de l'élasticité cutanée et par la formation de rides. Il peut être provoqué notamment par des troubles pathologiques de la kératinisation, par le vieillissement ou par l'exposition excessive au soleil ou à divers agents extérieurs (détergents usuels, savons, solvants, atmosphère sèche, etc...). Ce phénomène peut affecter toutes les parties du corps, et particulièrement le visage, le cou et les mains.

En outre, certains dérivés de formule (I) peuvent former dans l'eau ou dans les solvants aqueux des structures vésiculaires capables de piéger et de retenir des substances hydrophobes ou hydrophiles, et peuvent être utilisés sous cette forme comme véhicules d'ingrédients actifs lipophiles ou hydrophiles, notamment dans des compositions cosmétiques, hygiéniques ou pharmaceutiques.

La présente invention a donc également pour objet une composition cosmétique, hygiénique ou pharmaceutique caractérisée par le fait qu'elle comprend comme ingrédient actif au moins un dérivé de formule I, tel que défini précédemment, ou un sel correspondant, dans un véhicule compatible avec l'application chez l'homme sur la peau et/ou sur les cheveux et/ou avec l'application dans les soins d'hygiène bucco-dentaire.

Dans les compositions de l'invention, les dérivés de formule I, sont présents à une concentration pouvant aller de 0,05 à 20 %, et de préférence de 0,5 à 10% en poids, par rapport au poids total de la composition.

Les compositions de l'invention sont notamment des solutions du type lotions, moussantes ou non ; des émulsions de consistance liquide ou semi-liquide du type laits, obtenues par dispersion d'une phase grasse dans une phase aqueuse ou inversement ; des suspensions ou émulsions de consistance molle du type crème ou pommade ; des gels ; ou encore des préparations solides telles que des sticks, des pains de nettoyage ou des tampons imprégnés.

Les véhicules présents dans les compositions de l'invention sont les véhicules classiques utilisés dans ce genre de composition. Il s'agit par exemple de l'eau et des solvants organiques compatibles avec l'application cutanée tels que par exemple l'acétone, l'alcool isopropylique, l'alcool éthylique, les triglycérides d'acides gras en C₆-C₂₄, les éthers de glycols tels que les éthers d'alkyle inférieur de mono-ou di-alkylène glycols, l'alkylène ayant par exemple 2 à 4 atomes de carbone. On peut également utiliser comme solvants les esters de polyalkylèneglycol et d'acides à chaîne courte en C₁-C₄, ou encore des silicones volatiles.

Les compositions peuvent aussi contenir, le cas échéant, des corps gras, notamment des huiles naturelles ou synthétiques.

Les compositions de l'invention peuvent également renfermer des agents épaississants ou gélifiants tels que la cellulose ou des dérivés de cellulose, par exemple à raison de 0,5 à 20% en poids par rapport au poids total de la composition. Les agents épaississants peuvent encore être constitués par des polymères acryliques, des alginates, des gommes, telles que la gomme de xanthane, de guar, de caroube, la gomme arabique ou bien des polyéthylèneglycols, des bentonites et des montmorillonites.

Les compositions de l'invention peuvent en outre contenir d'autres agents hydratants ou humectants connus, tels que la glycérine, la triacétine, ou plus généralement d'autres ingrédients actifs tels que des agents actifs contre le vieillissement de la peau.

Les compositions de l'invention peuvent également contenir des adjuvants usuels tels que des agents anti-oxydants, des agents conservateurs, des parfums, des colorants, etc...

Parmi les anti-oxydants, on citera les tert-butylhydroxyquinone, le butylhydroxyanisole, le butylhydroxytoluène et l'alpha-tocophérol et ses dérivés.

Les compositions pour la peau se présentent notamment sous la forme de crèmes, de laits, de gels, de lotions éventuellement épaissies, de solutions moussantes pour la douche ou le bain, de tampons imprégnés, de pommades, de sticks ou sous forme de pains ou de masques hydratants.

Les compositions pour cheveux sont notamment des shampooings dans lesquels le dérivé de formule I ou le sel correspondant, peut être associé à d'autres agents tensio-actifs tels que les tensio-actifs anioniques, cationiques, non ioniques, amphotères ou leurs mélanges.

Les compositions de l'invention peuvent également se présenter sous la forme de solutions ou de dispersions contenant des dérivés de formule I tels que définis ci-dessus, sous la forme vésiculaire, les vésicules pouvant alors servir d'agents d'encapsulation pour des ingrédients actifs lipophiles ou hydrophiles tels que l'acide rétinoïque, des agents filtrant les rayons ultraviolets, ou encore d'autres agents hydratants.

Toutes ces compositions sont préparées selon les méthodes usuelles.

Les compositions de l'invention peuvent être également des compositions pour les soins d'hygiène bucco-dentaire, dans lesquelles les composés de formule I et/ou leurs sels jouent notamment le rôle d'agents nettoyants et moussants. L'un des intérêts de l'utilisation de ces composés dans les compositions de ce type est leur absence de nocivité. Ces compositions sont par exemple des bains de bouche ou des dentifrices. Les dentifrices peuvent se présenter sous la forme de pâtes ou sous la forme de gels transparents. Ils contiennent, outre au moins un agent tensioactif selon l'invention, au moins un matériau minéral pulvérulent jouant le rôle d'agent de polissage, par exemple une poudre d'alumine ou de silice. L'agent de polissage représente par exemple de 10 à 80 % en poids par rapport au poids total de la composition. Les compositions dentifrices peuvent contenir en outre des agents de cohésion comme des gommes naturelles ou des épaississants synthétiques (notamment des dérivés de cellulose tels que la méthylcellulose, les hydroxyalkylcelluloses ou le sel de sodium de la carboxyméthylcellulose). Ces agents de cohésion peuvent être incorporés par exemple dans une proportion pondérale pouvant aller jusqu'à 10 %.

Les compositions dentifrices peuvent êgalement contenir des agents de texture et de consistance, dans une proportion pondérale pouvant aller par exemple jusqu'à 60 %. On utilise par exemple comme agent de texture le sorbitol, qui a par ailleurs des propriétés édulcorantes et anti-bactériennes.

Les compositions sous forme de bains de bouche sont des compositions liquides qui consistent essentiellement en une solution aqueuse d'un agent tensioactif nettoyant et moussant. Outre l'agent tensioactif selon l'invention ces compositions peuvent encore contenir un ou plusieurs ingrédients usuels tels que des agents épaississants.

Les compositions d'hygiène bucco-dentaire, quelle que soit leur présentation, peuvent contenir en outre, en proportion efficace, au moins un ingrédient usuel choisi parmi les édulcorants, les agents aromatisants, les agents antibactériéns, les sources d'ions fluorure, les agents conservateurs, etc...

La préparation des compositions dentifrices ou des bains de bouche, ainsi que les ingrédients utilisables, sont bien connus et décrits par exemple dans les ouvrages suivants : Handbook of Cosmetic Science, H.W. Hibbott Ed. Pergamon Press (Oxford, Londres, Nex-York, Paris), et Harry's Cosmeticology, Leonard Hill Books (Londres).

L'invention a également pour objet l'utilisation d'un dérivé de formule (I) tel que défini précédemment / ou d'un sel correspondant, comme agent tensioactif et / ou comme agent hydratant dans la préparation d'une composition cosmétique, hygiénique ou pharmaceutique destinée au traitement ou au soin des peaux sèches.

L'invention a en outre pour objet un procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie précédemment.

L'application des compositions de l'invention est effectuée selon les méthodes usuelles.

Le procédé de traitement cosmétique de l'invention est applicable en complément au traitement de dermatoses sèches, d'ichtiosis, de xéroses, etc....

L'invention a également pour objet, à titre de produits nouveaux, les amides dipeptidiques de formule générale (II) :

R'CO - NHCH₂CONH - CH(CH₂OH) - COOH (II)

dans laquelle R' représente un groupement alkyle linéaire ou ramifié, éventuellement insaturé, ayant de 7 à 12 ou de 14 à 17 atomes de carbone, ainsi que des sels des composés de formule (II) et les mélanges des composés de formule (II) et/ou de leurs sels.

On voit que les composés de formule (II) correspondent aux composés de formule (I) à l'exception de la N-tétradécanoyl-glycyl-sérine et des ses sels. Le groupement R' de la formule (II) peut donc avoir les mêmes significations que le groupement R de la formule (I), à l'exception d'un groupement en C₁₃.

L'invention a également pour objet un procédé de préparation des composés de formule (II).

Ce procédé consiste principalement à faire réagir un sel de sérine avec un composé de formule (III) :

R'CO - NHCH₂ - CO - O - CO - OR'' (III)

dans laquelle R' est défini comme précédemment et R'' représente un groupement éthyle ou isopropyle.

La réaction peut être effectuée notamment dans un milieu solvant telle que des mélanges eau/tétrahydrofuranne ou eau/N,N-diméthylformamide. On peut opérer à température ambiante.

Pour isoler le produit de formule (II) (forme acide) on peut soit évaporer les solvants, soit faire une extraction avec un solvant organique tel que l'acétate d'éthyle. On peut ensuite purifier le produit obtenu par recristallisation, par exemple dans un hydrocarbure liquide à la température ambiante.

Le sel de départ est par exemple un sel de métal alcalin ou un sel d'amine tel qu'un sel de triéthylamine.

Si désiré, on transforme, selon les méthodes connues, le composé de formule (II) en sel correspondant.

Des détails supplémentaires sont donnés dans la partie expérimentale ci-après.

Les exemples suivants illustrent l'invention sans toutefois la limiter.

### EXEMPLES DE PREPARATION

Tous les lipodipeptides correspondant à la formule (I) sont synthétisés selon le mode opératoire suivant :

### A. Préparation de l'alcanoyl-glycine, de formule IV,

R - CO - NH - CH₂ - COOH (IV)

R possédant les mêmes significations que dans la formule (I).

A 1 équivalent de l'acide R - COOH choisi, dissous à une concentration de 25 % (poids/volume) dans Te tétrahydrofuranne, on ajoute 1,05 équivalent de triéthylamine. On agite pendant 1 heure à température ambiante puis verse dans un ballon contenant un équivalent de chloroformiate d'éthyle à 10 % dans le tétrahydrofuranne, à une température de -10 °C. On laisse le milieu réactionnel pendant 3 heures à température ambiante, puis on filtre et verse le filtrat dans une solution de sel de sodium de la glycine, obtenue préalablement par addition d'un équivalent d'hydroxyde de sodium (solution aqueuse à 10 %) à 1 équivalent de glycine, en prenant soin de maintenir le pH supérieur à 9. On agite le mélange pendant 3 heures à température ambiante tout en continuant de maintenir un pH supérieur à 9, puis on verse de l'acide chlorhydrique concentré jusqu'à obtention d'un pH égal à 2. On extrait par un mélange eau/acétate d'éthyle (1 : 1). Les phases organiques sont séchées et concentrées sous pression réduite, puis le résidu est recristallisé dans un solvant choisi parmi l'heptane, l'éther iso-propylique, le tert-butylméthyléther, l'acétate d'éthyle ou leurs mélanges.

### B. Préparation du dérivé de formule (I) (forme acide)

A 1 équivalent d'alcanoyl glycine obtenu au stade précédent, mis en solution dans 30 % de tétrahydrofuranne, on ajoute 1,05 équivalent de triéthylamine. On agite 1 heure à température ambiante, puis on verse le milieu réactionnel dans une solution de chloroformiate d'éthyle (ou de chloroformiate d'isopropyle) (1 éq.) à 10 % dans le tétrahydrofuranne, on mantient la température à - 10°C. Le mélange réactionnel est agité pendant 3 heures à température ambiante, puis filtré, et le filtrat est versé dans une solution de sel de sodium de sérine préparée par ajout d'un équivalent d'hydroxyde de sodium (solution aqueuse à 10 %) à un équivalent de sérine, tout en maintenant le pH supérieur à 9.

Le milieu réactionnel est ensuite laissé à température ambiante sous agitation pendant 3 heures (en maintenant un pH supérieur à 9), puis acidifié par de l'acide chlorhydrique concentré jusqu'à l'obtention d'un pH égal à 2. On extrait à l'aide d'un mélange eau/acétate d'éthyle (1 : 1). Les phases organiques sont réunies, séchées et concentrées sous pression réduite. Le résidu est recristallisé dans un solvant choisi parmi l'eau, l'heptane, l'éther isopropylique, le tert-butylméthyléther, l'acétate d'éthyle ou leurs mélanges.

### C. Préparation des sels correspondants (par exemple sel de sodium ou de triéthanolamine).

Les sels sont obtenus par une méthode connue consistant à additionner 1 équivalent d'hydroxyde de sodium (solution aqueuse à 10 %), ou 1 équivalent de triéthanolamine, à une solution à 5 % de l'acide correspondant dans un mélange isopropanol/eau ( 2 : 1) à une température pouvant varier par exemple entre 20 et 70 °C. La solution est ensuite concentrée à sec pour obtenir le sel attendu.

### EXEMPLE 1 : Préparation de la N-dodécanoyl-glycyl-(D,L) sérine

Ce produit est obtenu selon le procédé décrit ci-dessus et se présente sous forme d'une poudre blanche. Il est recristallisé dans un mélange eau/acétate d'éthyle.
F = 133°C

| Analyse élémentaire C₁₇H₃₂N₂O₅, 1H₂O ; M = 362,5 | | |
|---|---|---|
| | C | H |
| Calc.% | 56,32 | 9,45 |
| Tr.% | 55,88 | 8,95 |

Le spectre R.M.N du ¹³C est conforme à la structure indiquée.

### EXEMPLE 2 : Préparation de la N-oléoyl-glycyl-(DL) sérine

Ce produit est préparé de façon analogue à celle décrite à l'exemple 1, et se présente sous forme de poudre blanche. Il est recristallisé dans un mélange eau/acétate d'éthyle.
F : 87°C

| Analyse élémentaire : C₂₃H₄₂N₂O₅ ; M = 426,6 | | | |
|---|---|---|---|
| | C | H | N |
| Calc.% | 64,76 | 9,92 | 6,57 |
| Tr.% | 64,72 | 9,94 | 6,49 |

Le sepctre de R.M.N. du ¹³C est conforme à la structure indiquée.

### EXEMPLE 3 : Préparation de la N-linoléoyl-glycyl-(D,L) sérine.

Ce produit est obtenu de façon analogue, et se présente sous forme d'un solide collant jaune pâle.

| Analyse élémentaire : C₂₃H₄₀N₂O₅ ; M = 424,6 | | | |
|---|---|---|---|
| | C | H | N |
| Calc.% | 65,06 | 9,50 | 6,60 |
| Tr.% | 65,20 | 9,61 | 6,44 |

Le spectre de R.M.N du ¹³C est conforme à la structure indiquée.

### EXEMPLE 4 : Préparation du sel de triéthanolamine de la N-linoléoyl-glycyl-(D,L) sérine

Ce sel est préparé conformément au procédé décrit ci-dessus, et se présente sous la forme d'une pâte jaune.

| Analyse élémentaire : C₂₉H₅₅N₃O₈, 0,5 H₂O ; M = 582,8 | | | |
|---|---|---|---|
| | C | H | N |
| Calc.% | 59,77 | 9,68 | 7,20 |
| Tr.% | 59,81 | 9,71 | 7,00 |

### EXEMPLE 5 : Préparation du sel de sodium de la N-dodécanoyl-glycyl-(D,L) sérine

Ce sel est préparé de façon analogue, et se présente sous la forme d'un solide blanc.
F = 167°C

| Analyse élémentaire : C₁₇H₃₁N₂NaO₅, 0,5 H₂O ; M = 375,5 | | |
|---|---|---|
| | C | H |
| Calc.% | 54,37 | 8,59 |
| Tr.% | 54,93 | 8,80 |

### EXEMPLE 6 : Préparation du sel de sodium de la N-oléoyl-glycyl-(D,L) sérine

Ce sel est préparé selon un procédé analogue, et se présente sous la forme d'un solide blanc.
F = 170°C

| Analyse élémentaire : C₂₃H₄₁N₂NaO₅, 0,5H₂O ; M = 457,6 | | | |
|---|---|---|---|
| | C | H | N |
| Calc.% | 60,36 | 9,25 | 5,02 |
| Tr.% | 60,47 | 9,50 | 4,60 |

### EXEMPLE 7 : Préparation du sel de triéthanolamine de N-dodécanoyl- glycyl-(D,L) sérine

Ce sel est préparé selon un procédé analogue, et se présente sous la forme d'une pâte marron clair.

| Analyse élémentaire : C₂₃H₄₇N₃O₈, 2H₂O ; M = 529,6 | | | |
|---|---|---|---|
| | C | H | N |
| Calc.% | 52,16 | 9,70 | 7,93 |
| Tr.% | 51,62 | 9,29 | 7,51 |

### EXEMPLE 8 : Préparation de la N-octanoyl-glycyl-(D,L) sérine

Ce produit est obtenu selon un procédé décrit analogue. Il est recristallisé dans un mélange acétate d'éthyle/méthanol.
F = 97°C

| Analyse élémentaire : C₁₃H₂₄N₂O₅ ; M = 288,4 | | | |
|---|---|---|---|
| | C | H | N |
| Calc.% | 54,15 | 8,39 | 9,72 |
| Tr.% | 54,14 | 8,40 | 9,68 |

Le spectre R.M.N ¹³C est conforme à la structure indiquée.

### EXEMPLES DE COMPOSITIONS COSMETIQUES

### EXEMPLE A : Lotion

On a préparé, par mélange des ingrédients ci-après une lotion démaquillante à activité hydratante.

| | |
|---|---|
| N-dodécanoyl-glycyl-(D,L) sérine | 2,00 g |
| Anti-oxydant | 0,05 g |
| Conservateur | 0,30 g |
| Isopropanol | 40,00 g |
| Eau, q.s.p | 100,00 g |

On procède au démaquillage du visage à l'aide d'une éponge mouillée imbibée avec cette lotion.

### EXEMPLE B : Gel

On a préparé, par mélange des ingrédients ci-après, une composition sous forme de gel à activité hydratante.

| | |
|---|---|
| N-dodécanoyl-glycyl-(D,L) sérine | 2,00 g |
| Klucel H* | 1,00 g |
| Isopropanol | 40,00 g |
| Conservateur | 0,3 g |
| Anti-oxydant | 0,05 g |
| Eau, q.s.p | 100,00 g |

| | |
|---|---|
| * KLUCEL H est la dénomination commerciale d' hydroxypropyl cellulose vendue par la Société HERCULES. | |

On applique ce gel, de préférence le soir, sur le visage et le cou.

### EXEMPLE C : Crème de soins (émulsion H/E) pour la peau.

La composition de cette crème est la suivante :

| | |
|---|---|
| Sel de sodium de N-dodécanoyl-glycyl-(D,L) sérine | 3,00 g |
| Stéarate de glycérol | 2,00 g |
| TWEEN 60 | 1,00 g |
| Alcool cétylique | 0,50 g |
| Acide stéarique | 1,40 g |
| Triéthanolamine | 0,70 g |
| CARBOPOL 940 (neutralisé par la triéthanolamine) | 0,40 g |
| Fraction liquide de graisse de karité | 12,00 g |
| Perhydrosqualène de synthèse | 12,00 g |
| Anti-oxydant | 0,05 g |
| Parfum | 0,50 g |
| Conservateur | 0,30 g |
| Eau, q.s.p | 100,00 g |

Cette crème est préparée de la façon suivante :
On ajoute le CARBOPOL 940 neutralisé par la triéthanolamine à une partie de l'eau (85 à 90 %) et on chauffe à 75-80°C. On ajoute alors, en agitant, la phase grasse (stéarate de glycérol, TWEEN 60, acide stéarique, alcool cétylique, fraction liquide de graisse de karité, perhydrosqualène, anti-oxydant) portée à la même température, dans laquelle on a ajouté en dernier lieu la triéthanolamine. Après 10 min d'agitation, on ajoute le dérivé dipeptidique et le conservateur préalablement versés dans le restant de l'eau. Au bout de 10 min supplémentaires, on ajoute le parfum, puis on arrête l'agitation et on refroidit jusqu'à la température ambiante.

On rappelle que le TWEEN 60 est la dénomination commerciale d'un monostéarate de Sorbitan, polyoxyéthyléné à 20 moles d'oxyde d'éthylène, commercialisé par ICI AMERICAS.

CARPOBOL 940 est la dénomination commerciale d'un polymère acrylique réticulé par un agent polyfonctionnel, commercialisé par la Société GOODRICH.

On utilise cette crème pour l'hydratation de la peau.

### EXEMPLE D : Shampooing

Ce shampooing a la composition suivante :

| | |
|---|---|
| Sel de sodium de N-dodécanoyl-glycyl-(D,L) sérine | 10,00 g |
| KLUCEL H | 1,00 g |
| Parfum | 0,50 g |
| Conservateur | 0,30 g |
| Eau, q.s.p | 100,00 g |

Pour préparer ce shampooing, le sel de sodium de la N-dodécanoyl-glycyl-(D,L) sérine est dissous à 60°C dans de l'eau jusqu'à obtention d'une solution limpide. On ajoute à ce milieu une solution aqueuse épaissie par le KLUCEL H, ayant également une température de 60°C. On ajoute le parfum et le conservateur puis on ajuste avec de l'eau le poids final et on laisse refroidir.

### EXEMPLE E :

On prépare un gel destiné à l'hygiène bucco-dentaire de la composition suivante (% en poids) :

| Ingrédients | % |
|---|---|
| Silice en poudre vendue par la Société DEGUSSA sous la dénomination commerciale "Sident 9" | 12 |
| Silice en poudre vendue par la Société DEGUSSA sous la dénomination commerciale "Sident 22 S" | 7 |
| Carboxyméthyl cellulose | 0,5 |
| Sorbitol à 70 % de matière active | 66 |
| Dodécyloxycarbonyl glycyl sérine neutralisé par la soude jusqu'à pH = 7 | 1 |
| Parahydroxybenzoate de méthyle | 0,2 |
| Monofluoro phosphate de sodium | 0,8 |
| Agent de sapidité qs | |
| Colorant qs | |
| Edulcorant qs | |
| Eau qsp | 100 |

### ETUDE DU POUVOIR MOUSSANT

Cette étude a été effectuée avec des solutions aqueuses à 5 % (poids/volume) du composé de l'exemple 5. Le produit de comparaison est une solution à 5 % du composé A (sel de sodium du N-dodécanoyl-glycyl-glycine).

Le test consiste à développer la mousse à l'aide d'un pinceau en soies de porc naturelles (longueur = 18 mm) se déplaçant dans un récipient gradué cylindrique en plexiglas (diamètre intérieur 35 mm). Ce récipient est animé d'un mouvement circulaire uniforme inverse de celui décrit par le pinceau ; cela permet une bonne répartition de la mousse dans le récipient gradué malgré les faibles quantités développées (vitesse du pinceau 100 t/min, vitesse du récipient 10 t/min).

La manipulation s'effectue avec 5 ml de chaque solution étudiée, additionnée de 6 mg de sebum naturel féminin.

On relève les hauteurs de mousse après une, deux et trois min d'agitation.

Les résultats sont résumés dans le tableau I.

**TABLEAU I**

| Composé | Hauteur de mousse (cm) au bout d'un temps de | | |
|---|---|---|---|
| | 60 s | 120 s | 180s |
| A | 11,25 | 13,75 | 20 |
| Ex. 5 | 12,5 | 21,25 | 28,75 |

### EFFETS HYDRATANTS SUR LA PEAU

Dans ce test, l'effet hydratant est évalué en mesurant la diminution de la perte en eau d'un stratum corneum humain préalablement délipidé. Le stratum corneum est délipidé pendant une heure avec un mélange dichlorométhane:méthanol (2:1).

On effectue ce test selon une méthode analogue à celle décrite par J.L. LEVEQUE et coll., J. Soc. Cosmet. Chem., 30, 333-343 (1979).

On opère de la façon suivante :
On mesure le flux d'eau (g/m².h) traversant un échantillon de stratum corneum situé au-dessus d'une réservoir d'eau. Grâce à un évaporimètre (Servomed, marque déposée), on enregistre les valeurs d'évaporation avant et après traitement, et on détermine ainsi l'éventuelle diminution de la perte insensible d'eau.

Le traitement consiste à appliquer sur l'échantillon de stratum corneum 10 »l d'une solution à 3 %, dans le mélange dichlorométhane:méthanol 2:1, du composé à étudier. Les mesures sont effectuées 3 heures après ce traitement.

On a comparé sur la base de ce test le composé de l'exemple 2 avec la N-oléoyl-glycyl-glycine (composé B), et avec le solvant (dichlorométhane : méhanol 2 : 1) seul.

Les résultats sont résumés dans le tableau II.

**TABLEAU II**

| Composé étudié | Diminution de la perte insensible d'eau |
|---|---|
| B | 6 % ± 3 |
| Ex.2 | 32 % ± 3 |
| Solvant | 6 % ± 3 |

## Revendications (Revendications pour l'(les) Etat(s) contractant(s) suivant(s): AT, BE, CH, DE, DK, FR, GB, GR, IT, LI, NL, PT, SE)

1. Utilisation comme agents tensioactifs et/ou comme agents hydratants, des amides dipeptidiques de formule générale (I)
RCO - NHCH₂CONH - CH(CH₂OH) - COOH (I)
dans laquelle R représente un groupement alkyle, linéaire ou ramifié, éventuellement insaturé, ayant de 7 à 17 atomes de carbone, ainsi que des sels des composés de formule (I), et des mélanges des composés de formule (I) et/ou de leurs sels.

2. Utilisation selon la revendication 1, comme agents tensioactifs ou hydratants dans des compositions cosmétiques, hygiéniques, pharmaceutiques ou alimentaires

3. Utilisation selon la revendication 1 ou 2, caractérisée par le fait que lesdits sels sont des sels métalliques ou des sels de cations organiques compatibles avec l'application recherchée.

4. Utilisation selon la revendication 2, caractérisée par le fait que lesdits sels sont choisis parmi les sels de sodium, de zinc, de magnésium et d'aluminium, et les sels cuivriques, et les sels d'ammonium quaternaires.

5. Utilisation selon la revendication 4, caractérisée par le fait que lesdits sels d'ammonium quaternaires sont des sels d'un cation de formule 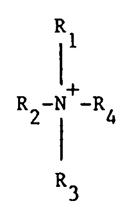 dans laquelle R₁, R₂, R₃ et R₄ représentent indépendamment un groupement -CH₃, -CH₂-C₆H₅ ou -CH₂-CH₂OH.

6. Utilisation selon l'une quelconque des revendications précédentes, caractérisée par le fait que R est choisi de façon que RCO-représente un groupement octanoyle, décanoyle, dodécanoyle, tétradécanoyle, oléoyle ou linoléoyle.

7. Utilisation d'au moins un dérivé de dipeptide, tel que défini dans l'une quelconque des revendications 1 à 6, comme agent tensioactif.

8. Utilisation d'un dérivé de dipeptide tel que défini dans l'une quelconque des revendications 1 à 6 comme ingrédient actif dans la préparation d'une composition cosmétique ou pharmaceutique destinée au traitement ou au soin des peaux sèches.

9. Composition cosmétique, hygiénique ou pharmaceutique, caractérisée par le fait qu'elle comprend comme agent tensioactif ou comme agent hydratant au moins un dérivé de dipeptide tel que défini dans l'une quelconque des revendications 1 à 6, dans un véhicule compatible avec l'administration sur la peau et/ou sur les cheveux, ou compatible avec l'application dans les soins d'hygiène bucco-dentaire.

10. Composition selon la revendication 9, caractérisée par le fait que la concentration dudit dérivé de dipeptide est dans la gamme de 0,05 à 20 % en poids, par rapport au poids total de la composition.

11. Composition selon la revendication 10, caractérisée par le fait que ladite concentration est dans la gamme 0,5 à 10 % en poids par rapport au poids total de la composition.

12. Procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie dans l'une quelconque des revendications 9 à 11.

13. Amides dipeptidiques de formule (I)
RCO - NHCH₂CONH - CH(CH₂OH) - COOH (I)
dans laquelle R représente un groupement alkyle, linéaire ou ramifié, éventuellement insaturé, ayant de 7 à 17 atomes de carbone,
ainsi que des sels des composés de formule (I), et des mélanges des composés de formule (I) et/ou de leurs sels,
à l'exception des composés de formule (I) pour lesquels R est un groupement en C₁₃.

14. Amides selon la revendication 13 ou sels correspondants, caractérisés par le fait que R est choisi de façon que RCO- représente un groupement octanoyle, décanoyle, dodécanoyle, tétradécanoyle, oléoyle ou linoléoyle.

15. Procédé de préparation d'un amide dipeptidique tel que défini dans l'une quelconque des revendications 13 et 14, caractérisé par le fait que l'on fait réagit un sel de sérine avec un composé de formule (III) :
R'CO - NHCH₂ - CO - O - CO - OR'' (III)
dans laquelle R' représente un groupement alkyle linéaire ou ramifié, éventuellement insaturé, ayant de 7 à 12 ou de 14 à 17 atomes de carbone, et R'' représente un groupement éthyle ou isopropyle ; et que, si désiré, on transforme le composé obtenu, de façon connue, en sel correspondant.

## Revendications (Revendications pour l'(les) Etat(s) contractant(s) suivant(s): ES)

1. Utilisation comme agents tensioactifs et/ou comme agents hydratants, des amides dipeptidiques de formule générale (I)
RCO - NHCH₂CONH - CH(CH₂OH) - COOH (I)
dans laquelle R représente un groupement alkyle, linéaire ou ramifié, éventuellement insaturé, ayant de 7 à 17 atomes de carbone, ainsi que des sels des composés de formule (I), et des mélanges des composés de formule (I) et/ou de leurs sels.

2. Utilisation selon la revendication 1, comme agents tensioactifs ou hydratants dans des compositions cosmétiques, hygiéniques, pharmaceutiques ou alimentaires

3. Utilisation selon la revendication 1 ou 2, caractérisée par le fait que lesdits sels sont des sels métalliques ou des sels de cations organiques compatibles avec l'application recherchée.

4. Utilisation selon la revendication 2, caractérisée par le fait que lesdits sels sont choisis parmi les sels de sodium, de zinc, de magnésium et d'aluminium, et les sels cuivriques, et les sels d'ammonium quaternaires.

5. Utilisation selon la revendication 4, caractérisée par le fait que lesdits sels d'ammonium quaternaires sont des sels d'un cation de formule 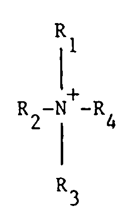 dans laquelle R₁, R₂, R₃ et R₄ représentent indépendamment un groupement -CH₃, -CH₂-C₆H₅ ou -CH₂-CH₂OH.

6. Utilisation selon l'une quelconque des revendications précédentes, caractérisée par le fait que R est choisi de façon que RCO-représente un groupement octanoyle, décanoyle, dodécanoyle, tétradécanoyle, oléoyle ou linoléoyle.

7. Utilisation d'au moins un dérivé de dipeptide, tel que défini dans l'une quelconque des revendications 1 à 6, comme agent tensioactif.

8. Utilisation d'un dérivé de dipeptide tel que défini dans l'une quelconque des revendications 1 à 6 comme ingrédient actif dans la préparation d'une composition cosmétique ou pharmaceutique destinée au traitement ou au soin des peaux sèches.

9. Composition cosmétique ou hygiénique, caractérisée par le fait qu'elle comprend comme agent tensioactif ou comme agent hydratant au moins un dérivé de dipeptide tel que défini dans l'une quelconque des revendications 1 à 6, dans un véhicule compatible avec l'administration sur la peau et/ou sur les cheveux, ou compatible avec l'application dans les soins d'hygiène bucco-dentaire.

10. Composition selon la revendication 9, caractérisée par le fait que la concentration dudit dérivé de dipeptide est dans la gamme de 0,05 à 20 % en poids par rapport au poids total de la composition.

11. Composition selon la revendication 10, caractérisée par le fait que ladite concentration est dans la gamme 0,5 à 10 % en poids par rapport au poids total de la composition.

12. Procédé de traitement cosmétique destiné notamment à améliorer l'aspect et l'élasticité de la peau des personnes à peau sèche ou destiné à prévenir l'apparition des troubles esthétiques provoqués par ce phénomène de peau sèche, caractérisé par le fait que l'on applique sur la peau des parties du corps concernées y compris éventuellement le cuir chevelu, une composition cosmétique telle que définie dans l'une quelconque des revendications 9 à 11.

13. Procédé de préparation d'un amide dipeptidique de formule (I)
RCO - NHCH₂CONH - CH(CH₂OH) - COOH (I)
dans laquelle R représente un groupement alkyle, linéaire ou ramifié, éventuellement insaturé, ayant de 7 à 17 atomes de carbone,
ainsi que des sels des composés de formule (I), et des mélanges des composés de formule (I) et/ou de leurs sels,
à l'exception des composés de formule (I) pour lesquels R est un groupement en C₁₃, caractérisé pour le fait que l'on fait réagir un sel de sérine avec un composé de formule (III) :
R'CO - NHCH₂ - CO - O - CO - OR'' (III)
dans laquelle R' représente un groupement alkyle linéaire ou ramifié, éventuellement insaturé, ayant de 7 à 12 ou de 14 à 17 atomes de carbone, et R'' représente un groupement éthyle ou isopropyle ; et que, si désiré, on transforme le composé obtenu, de façon connue, en sel correspondant.

14. Procédé selon la revendication 13, caractérisé par le fait que R' est choisi de façon que R'CO- représente un groupement octanoyle, décanoyle, dodécanoyle, tétradécanoyle, oléoyle ou linoléoyle.

## Claims (Claims for the following Contracting State(s): AT, BE, CH, DE, DK, FR, GB, GR, IT, LI, NL, PT, SE)

1. Use as surfactants and/or as hydrating agents of the dipeptide amides of general formula (I)
RCO - NHCH₂CONH - CH(CH₂OH) - COOH (I)
in which R represents an optionally unsaturated, linear or branched alkyl group having from 7 to 17 carbon atoms, as well as salts of the compounds of formula (I), and mixtures of the compounds of formula (I) and/or their salts.

2. Use according to Claim 1, as surfactants or hydrating agents in cosmetic, hygiene, pharmaceutical or food compositions.

3. Use according to Claim 1 or 2, characterized in that the said salts are metal salts or salts of organic cations which are compatible with the desired application.

4. Use according to Claim 2, characterized in that the said salts are chosen from sodium, zinc, magnesium and aluminium salts, cupric salts and quaternary ammonium salts.

5. Use according to Claim 4, characterized in that the said quaternary ammonium salts are salts of a cation of formula 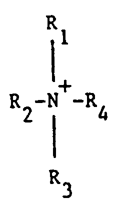 in which R₁, R₂, R₃ and R₄ independently represent a -CH₃, -CH₂-C₆H₅ or -CH₂-CH₂OH group.

6. Use according to any one of the preceding claims, characterized in that R is chosen such that RCO- represents an octanoyl, decanoyl, dodecanoyl, tetradecanoyl, oleoyl or linoleoyl group.

7. Use of at least one dipeptide derivative as defined in any one of Claims 1 to 6, as a surfactant.

8. Use of a dipeptide derivative as defined in any one of Claims 1 to 6, as an active ingredient in the preparation of a cosmetic or pharmaceutical composition intended for the treatment or care of dry skins.

9. Cosmetic, hygiene or pharmaceutical composition, characterized in that it comprises as surfactant or as hydrating agent at least one dipeptide derivative as defined in any one of Claims 1 to 6, in a vehicle compatible with administration to the skin and/or to the hair, or compatible with application in dentibuccal hygiene care.

10. Composition according to Claim 9, characterized in that the concentration of the said dipeptide derivative is within the range 0.05 to 20% by weight relative to the total weight of the composition.

11. Composition according to Claim 10, characterized in that the said concentration is within the range 0.5 to 10% by weight relative to the total weight of the composition.

12. Cosmetic treatment process intended, in particular, for improving the appearance and elasticity of the skin of individuals having dry skin, or intended for preventing the onset of problems of unsightly appearance caused by this phenomenon of dry skin, characterized in that a cosmetic composition as defined in any one of Claims 9 to 11 is applied to the skin of the parts of the body affected, including, where appropriate, the scalp.

13. Dipeptide amides of formula (I)
RCO - NHCH₂CONH - CH(CH₂OH) - COOH (I)
in which R represents an optionally unsaturated, linear or branched alkyl group having from 7 to 17 carbon atoms, as well as salts of the compounds of formula (I), and mixtures of the compounds of formula (I) and/or their salts,
with the exception of the compounds of formula (I) for which R is a C₁₃ group.

14. Amides according to Claim 13 or corresponding salts, characterized in that R is chosen such that RCO-represents an octanoyl, decanoyl, dodecanoyl, tetradecanoyl, oleoyl or linoleoyl group.

15. Process for preparing a dipeptide wade as defined in either of Claims 13 and 14, characterized in that a serine salt is reacted with a compound of formula (III):
R'CO - NHCH₂ - CO - O - CO - OR'' (III)
in which R' represents an optionally unsaturated, linear or branched alkyl group having from 7 to 12 or from 14 to 17 carbon atoms, and R'' represents an ethyl or isopropyl group; and in that, if so desired, the compound obtained is converted in a known manner to a corresponding salt.

## Claims (Claims for the following Contracting State(s): ES)

1. Use as surfactants and/or as hydrating agents of the dipeptide amides of general formula (I)
RCO - NHCH₂CONH - CH(CH₂OH) - COOH (I)
in which R represents an optionally unsaturated, linear or branched alkyl group having from 7 to 17 carbon atoms, as well as salts of the compounds of formula (I), and mixtures of the compounds of formula (I) and/or their salts.

2. Use according to Claim 1, as surfactants or hydrating agents in cosmetic, hygiene, pharmaceutical or food compositions.

3. Use according to Claim 1 or 2, characterized in that the said salts are metal salts or salts of organic cations which are compatible with the desired application.

4. Use according to Claim 2, characterized in that the said salts are chosen from sodium, zinc, magnesium and aluminium salts, cupric salts and quaternary ammonium salts.

5. Use according to Claim 4, characterized in that the said quaternary ammonium salts are salts of a cation of formula 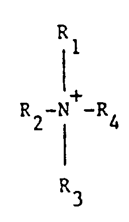 in which R₁, R₂, R₃ and R₄ independently represent a -CH₃, -CH₂-C₆H₅ or -CH₂-CH₂OH group.

6. Use according to any one of the preceding claims, characterized in that R is chosen such that RCO- represents an octanoyl, decanoyl, dodecanoyl, tetradecanoyl, oleoyl or linoleoyl group.

7. Use of at least one dipeptide derivative as defined in any one of Claims 1 to 6, as a surfactant.

8. Use of a dipeptide derivative as defined in any one of Claims 1 to 6, as an active ingredient in the preparation of a cosmetic or pharmaceutical composition intended for the treatment or care of dry skins.

9. Cosmetic or hygiene composition, characterized in that it comprises as surfactant or as hydrating agent at least one dipeptide derivative as defined in any one of Claims 1 to 6, in a vehicle compatible with administration to the skin and/or to the hair, or compatible with application in dentibuccal hygiene care.

10. Composition according to Claim 9, characterized in that the concentration of the said dipeptide derivative is within the range 0.05 to 20% by weight relative to the total weight of the composition.

11. Composition according to Claim 10, characterized in that the said concentration is within the range 0.5 to 10% by weight relative to the total weight of the composition.

12. Cosmetic treatment process intended, in particular, for improving the appearance and elasticity of the skin of individuals having dry skin, or intended for preventing the onset of problems of unsightly appearance caused by this phenomenon of dry skin, characterized in that a cosmetic composition as defined in any one of Claims 9 to 11 is applied to the skin of the parts of the body affected, including, where appropriate, the scalp.

13. Process for preparing a dipeptide amide of formula (I)
RCO - NHCH₂CONH - CH(CH₂OH) - COOH (I)
in which R represents an optionally unsaturated, linear or branched alkyl group having from 7 to 17 carbon atoms, as well as salts of the compounds of formula (I), and mixtures of the compounds of formula (I) and/or their salts,
with the exception of the compounds of formula (I) for which R is a C₁₃ group, characterized in that a serine salt is reacted with a compound of formula (III):
R'CO - NHCH₂ - CO - O - CO - OR'' (III)
in which R' represents an optionally unsaturated, linear or branched alkyl group having from 7 to 12 or from 14 to 17 carbon atoms, and R'' represents an ethyl or isopropyl group; and in that, if so desired, the compound obtained is converted in a known manner to a corresponding salt.

14. Process according to Claim 13, characterized in that R' is chosen such that R'CO- represents an octanoyl, decanoyl, dodecanoyl, tetradecanoyl, oleoyl or linoleoyl group.

## Patentansprüche (Patentansprüche für folgende(n) Vertragsstaat(en): AT, BE, CH, DE, DK, FR, GB, GR, IT, LI, NL, PT, SE)

1. Verwendung von Dipeptidamiden der folgenden allgemeinen Formel (I) als Tenside und/oder Hydratisierungsmittel
RCO - NHCH₂CONH - CH(CH₂OH) - COOH (I)
worin R einen geradkettigen oder verzweigtkettigen, gegebenenfalls ungesättigten Alkylrest mit 7 bis 17 Kohlenstoffatomen darstellt,
sowie Salzen von Verbindungen gemäß Formel (I) und Mischungen aus Verbindungen gemäß Formel (I) und/oder deren Salzen.

2. Verwendung (von Stoffen) gemäß Anspruch 1 als Tenside oder Hydratisierungsmittel in kosmetischen oder hygienischen Zubereitungen Arznei- oder Lebensmitteln.

3. Verwendung gemäß einem der Ansprüche 1 oder 2, dadurch gekennzeichnet, daß die Salze in Form von Metallsalzen oder organischen Kationensalzen vorliegen, welche jeweils mit der untersuchten (gewünschten) Applikation verträglich sind.

4. Verwendung gemäß Anspruch 2, dadurch gekennzeichnet, daß die Salze aus der Reihe Natriumsalze, Zinksalze, Magnesium- und Aluminiumsalze, Kupfersalze sowie quaternäre Ammoniumsalze ausgewählt wurden.

5. Verwendung gemäß Anspruch 4, dadurch gekennzeichnet, daß die quaternären Ammoniumsalze aus Salzen eines Kations der Formel 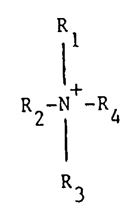 bestehen, worin R₁, R₂, R₃ und R₄ unabhängig voneinander die Gruppe -CH₃, -CH₂-C₆H₅ oder -CH₂-CH₂OH bedeuten.

6. Verwendung gemäß einem der vorstehenden Ansprüche, dadurch gekennzeichnet, daß R in der Weise ausgewählt wurde, daß RCO- eine Octanoyl-, Decanoyl-, Dodecanoyl-, Tetradecanoyl-, Oleoyl- oder Linoleoylgruppe darstellt.

7. Verwendung zumindest eines Dipeptidderivats gemäß Definition nach einem der Ansprüche 1 bis 6 als Tensid.

8. Verwendung eines Dipeptidderivats gemäß Definition nach einem der Ansprüche 1 bis 6 als aktiven Bestandteil bei der Herstellung einer kosmetischen Zubereitung oder eines Arzneimittels, welche jeweils zur Behandlung oder Pflege trockener Haut bestimmt sind.

9. Kosmetische oder hygienische Zubereitung oder Arzneimittel, dadurch gekennzeichnet, daß diese jeweils als Tensid oder als Hydratisierungsmittel mindestens ein Dipeptidderivat gemäß Definition nach einem der Ansprüche 1 bis 6 einen im Zusammenhang mit der Applikation auf der Haut und/oder auf den Haaren vertraglichen Träger aufweisen oder welche einen im Zusammenhang mit der Applikation zur Hygienepflege im Bereich des Mundes und der Zähne verträgliche Grundlage enthalten.

10. Zubereitung gemäß Anspruch 9, dadurch gekennzeichnet, daß die Konzentration an dem Dipeptidderivat im Bereich zwischen 0,05 und 20 Gew.-% in bezug auf das Gesamtgewicht der Zusammensetzung beträgt.

11. Zubereitung gemäß Anspruch 10, dadurch gekennzeichnet, daß die Konzentration in einem Bereich zwischen 0,5 und 10 Gew.-% in bezug auf das Gesamtgewicht der Zubereitung liegt.

12. Verfahren zur kosmetischen Behandlung, welche insbesondere zur Verbesserung des Aussehens und der Elastizität der Haut von Personen mit trockener Haut oder zur Vorbeugung des Aussehens bestimmt ist, welche durch ästhetische Probleme aufgrund des Phänomens der trockenen Haut verursacht werden, dadurch gekennzeichnet, daß auf der Haut an den betroffenen Körperstellen gegebenenfalls einschließlich der behaarten Kopfhaut eine kosmetische Zubereitung laut Definition gemäß einem der Ansprüche 9 bis 11 appliziert wird.

13. Dipeptidamide gemäß Formel (I)
RCO - NHCH₂CONH - CH(CH₂OH) - COOH (I)
worin R einen gegebenenfalls ungesättigten geradkettigen oder verzweigtkettigen Alkylrest mit 7 bis 17 Kohlenstoffatomen bedeutet,
sowie Salze der Verbindungen gemäß Formel (I) und Mischungen aus Verbindungen gemäß Formel (I) und/oder deren Salze,
mit Ausnahme von Verbindungen gemäß Formel (I), bei denen R eine C₁₃-Gruppierung bedeutet.

14. Amide gemäß Anspruch 13 oder die entsprechenden Salze, dadurch gekennzeichnet, daß R in der Weise ausgewählt wird, daß RCO- eine Octanoyl-, Decanoyl-, Dodecanoyl-, Tetradecanoyl-, Oleoyl- oder Linoleoylgruppe darstellt.

15. Verfahren zur Herstellung eines Dipeptidamids gemäß einem der Ansprüche 13 und 14, dadurch gekennzeichnet, daß man ein Serinsalz mit einer Verbindung gemäß Formel (III)
R'CO - NHCH₂ - CO - O - CO - OR'' (III)
reagieren läßt, worin R' einen gegebenenfalls ungesättigten geradkettigen oder verzweigtkettigen Alkylrest mit 7 bis 12 oder 14 bis 17 Kohlenstoffatomen bedeutet und R'' eine Ethyl- oder Isopropylgruppe darstellt, und daß gewünschtenfalls die erhaltene Verbindung in bekannter Weise zu dem entsprechenden Salz umgesetzt wird.

## Patentansprüche (Patentansprüche für folgende(n) Vertragsstaat(en): ES)

1. Verwendung von Dipeptidamiden der folgenden allgemeinen Formel (I )als Tenside und/oder Hydratisierungsmittel
RCO - NHCH₂CONH - CH(CH₂OH) - COOH (I)
worin R einen geradkettigen oder verzweigtkettigen, gegebenenfalls ungesättigten Alkylrest mit 7 bis 17 Kohlenstoffatomen darstellt,
sowie Salzen von Verbindungen gemäß Formel (I) und Mischungen aus Verbindungen gemäß Formel (I) und/oder deren Salzen.

2. Verwendung (von Stoffen) gemäß Anspruch 1 als Tenside oder Hydratisierungsmittel in kosmetischen oder hygienischen Zubereitungen, Arznei- oder Lebensmitteln.

3. Verwendung gemäß einem der Ansprüche 1 oder 2, dadurch gekennzeichnet, daß die Salze in Form von Metallsalzen oder organischen Kationensalzen vorliegen, welche jeweils mit der untersuchten (gewünschten) Applikation verträglich sind.

4. Verwendung gemäß Anspruch 2, dadurch gekennzeichnet, daß die Salze aus der Reihe Natriumsalze, Zinksalze, Magnesium- und Aluminiumsalze, Kupfersalze sowie quaternäre Ammoniumsalze ausgewählt wurden.

5. Verwendung gemäß Anspruch 4, dadurch gekennzeichnet, daß die quaternären Ammoniumsalze aus Salzen eines Kations der Formel 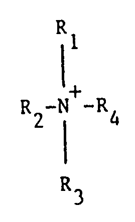 bestehen, worin R₁, R₂, R₃ und R₄ unabhängig voneinander die Gruppe -CH₃, -CH₂-C₆H₅ oder -CH₂-CH₂OH bedeuten.

6. Verwendung gemäß einem der vorstehenden Ansprüche, dadurch gekennzeichnet, daß R in der Weise ausgewählt wurde, daß RCO- eine Octanoyl-, Decanoyl-, Dodecanoyl-, Tetradecanoyl-, Oleoyl- oder Linoleoylgruppe darstellt.

7. Verwendung zumindest eines Dipeptidderivats gemäß Definition nach einem der Ansprüche 1 bis 6 als Tensid.

8. Verwendung eines Dipeptidderivats gemäß Definition nach einem der Ansprüche 1 bis 6 als aktiven Bestandteil bei der Herstellung einer kosmetischen Zubereitung oder eines Arzneimittels, welche jeweils zur Behandlung oder Pflege trockener Haut bestimmt sind.

9. Kosmetische oder hygienische Zubereitung oder Arzneimittel, dadurch gekennzeichnet, daß diese jeweils als Tensid oder als Hydratisierungsmittel mindestens ein Dipeptidderivat gemäß Definition nach einem der Ansprüche 1 bis 6 einen im Zusammenhang mit der Applikation auf der Haut und/oder auf den Haaren verträglichen Träger aufweisen oder welche einen im Zusammenhang mit der Applikation zur Hygienepflege im Bereich des Mundes und der Zähne verträgliche Grundlage enthalten.

10. Zubereitung gemäß Anspruch 9, dadurch gekennzeichnet, daß die Konzentration an dem Dipeptidderivat im Bereich zwischen 0,05 und 20 Gew.-% in bezug auf das Gesamtgewicht der Zusammensetzung beträgt.

11. Zubereitung gemäß Anspruch 10, dadurch gekennzeichnet, daß die Konzentration in einem Bereich zwischen 0,5 und 10 Gew.-% in bezug auf das Gesamtgewicht der Zubereitung liegt.

12. Verfahren zur kosmetischen Behandlung, welche insbesondere zur Verbesserung des Aussehens und der Elastizität der Haut von Personen mit trockener Haut oder zur Vorbeugung des Aussehens bestimmt ist, welche durch ästhetische Probleme aufgrund des Phänomens der trockenen Haut verursacht werden, dadurch gekennzeichnet, daß auf der Haut an den betroffenen Körperstellen gegebenenfalls einschließlich der behaarten Kopfhaut eine kosmetische Zubereitung laut Definition gemäß einem der Ansprüche 9 bis 11 appliziert wird.

13. Verfahren zur Herstellung eines Dipeptidamids gemäß Formel (I)
RCO - NHCH₂CONH - CH(CH₂OH) - COOH (I)
worin R einen gegebenenfalls ungesättigten geradkettigen oder verzweigtkettigen Alkylrest mit 7 bis 17 Kohlenstoffatomen bedeutet,
sowie Salze der Verbindungen gemäß Formel (I) und Mischungen aus Verbindungen gemäß Formel (I) und/oder deren Salze,
mit Ausnahme von Verbindungen gemäß Formel (I), bei denen R eine C₁₃-Gruppierung bedeutet,
dadurch gekennzeichnet, daß man ein Serinsalz mit einer Verbindung gemäß Formel (III)
R'CO - NHCH₂ - CO - O - CO - OR'' (III)
reagieren läßt, worin R' einen gegebenenfalls ungesättigten geradkettigen oder verzweigtkettigen Alkylrest mit 7 bis 12 oder 14 bis 17 Kohlenstoffatomen bedeutet und R'' eine Ethyl- oder Isopropylgruppe darstellt, und daß gewünschtenfalls die erhaltene Verbindung in bekannter Weise zu dem entsprechenden Salz umgesetzt wird.

14. Verfahren gemäß Anspruch 13, dadurch gekennzeichnet, daß R' in der Weise ausgewählt wird, daß R'CO- eine Octanoyl-, Decanoyl-, Dodecanoyl-, Tetradecanoyl-, Oleoyl- oder Linoleoylgruppe bedeutet.
